Europäisches Patentamt

European Patent Office (11) Publication number: **0 372 778**

Office européen des brevets **A1**

(12) **EUROPEAN PATENT APPLICATION**

(21) Application number: **89312281.2**

(22) Date of filing: **27.11.89**

(51) Int. Cl.5: **G03F 7/031, C07C 49/784, C07C 225/22**

(30) Priority: **01.12.88 US 278551**

(43) Date of publication of application:
**13.06.90 Bulletin 90/24**

(84) Designated Contracting States:
**AT BE CH DE ES FR GB GR IT LI LU NL SE**

(71) Applicant: **Polychrome Corporation**
**137 Alexander Street**
**Yonkers New York 10702(US)**

(72) Inventor: **Rowe, William**
**RR1 Box 103**
**Califon New Jersey(US)**
Inventor: **Shah, Ajay**
**241 W. Grand St.-C5**
**Elizabeth New Jersey(US)**

(74) Representative: **Lawrence, Peter Robin**
**Broughton et al**
**GILL JENNINGS & EVERY 53-64 Chancery**
**Lane**
**London WC2A 1HN(GB)**

(54) **Photoinitiator.**

(57) Photocurable compositions which comprise a light sensitive composition in combination with at least one initiator therefor are improved by the use of para-di(4-dimethylaminobenzoyl) benzene as the photoinitiator. The photocurable compositions can be used for various purposes including preparation of lithographic printing plates.

**EP 0 372 778 A1**

## PHOTOINITIATOR

Light sensitive resinous compositions are well known and are used for a wide variety of purposes including, for example, making lithographic printing plates, multicolor proofs, visual aids and printed circuits, and the like. These compositions generally comprise a photocurable component which may be a monomer, polymer or various combinations thereof together with an initiator. When irradiated with a suitable light source, free radicals are generated to cause polymerization, cross-linking or some combination thereof.

For example, lithographic printing plates have a coating of the light sensitive composition adhering to a suitable base sheet material such as an aluminum sheet. When the light sensitive coating is applied to the base sheet by the manufacturer, the plate is referred to as a "presensitized plate." When the coating is applied by the lithographic or trade platemaker, the plate is referred to as a "wipe-on" plate. As desired, the coated plate can be utilized to reproduce directly the image to which it is exposed, in which the case the plate is termed "positive-acting" or to reproduce a complimentary image, in which case the plate is designated "negative-acting". In either event, the image areas of the developed plate are relatively oleophilic and the non-image areas are relatively hydrophilic.

A popular photoinitiator in wide use today is benzophenone. Since it is particularly adapted for curing the surface of the photosensitive composition, the benzophenone is frequently employed in combination with an internal curing photoinitiator such as Michler's ketone. It has now been discovered that a new compound para-di(4-dimethylaminobenzoyl) benzene is an effective internal initiator, which in some formulations provides faster curing than Michler's ketone.

It is accordingly the object of this invention to provide a new photoinitiator and new light sensitive compositions containing such photoinitiator. This and other objects of the invention will become apparent to those skilled in this art from the following detailed description.

This invention relates to photoinitiators, photocurable compositions and their use and more particularly to photoinitiators, photocurable compositions comprising a light sensitive composition and photoinitiator in which the photoinitiator is para-di(dimethylaminobenzoyl) benzene.

The present invention is applicable to all photocurable compositions which comprise a light sensitive composition in combination with a photoinitiator. Such compositions are used, for example, to form lithographic printing plates, proofs for multi-colored printing, visual aids, printed circuits, UV curable 100%, non-volatile coatings and adhesives, UV curable printing inks and the like. For convenience only, the following description will focus on lithographic printing plates although it will be recognized that the invention is not limited thereto.

A lithographic printing plate generally has a coating of a light curable composition adhered to a suitable base sheet material. Any material known and commonly used as a lithographic base surface for printing plates can be used in the present invention and include those made from paper, fabric, synthetic resins, and especially in the case of long running dimensionally stable plates, from metals. Commonly used metals include steel, stainless steel, zinc, aluminum, copper, chromium, and for a variety of reasons well known in the art, aluminum in the form of a sheet. The base may have been treated in a number of ways known in the art to enhance its character as a lithographic surface such as, for instance, graining a metal plate either mechanically or chemically as described in U. S. Patents 2,882,153 and 2,882,154, to enable better bonding of the coating subsequently applied. An aluminum sheet can be electrolytically anodized to form a harder, more resistant and abrasion resistant surface as described in U.S. Patent 3,440,050 and British patent 1,235,863. If desired, an intermediate coating can be placed on the base coating to provide a firmer bond between the surface and the resulting photocurable composition as disclosed, for example, in U.S. Patents 2,946,683 and 3,160,506,

In general, the light curable composition can be any material or combination of materials which is polymerizable and/or cross-linkable through a free radical mechanism. Photocurable materials include acrylic or methacrylic acids esters of alcohols, polyhydric exemplified by trimethylolpropane triacrylate, pentaerythritol triacrylate and pentaerythritol pentacetate, dipentaerythritol hexamethacetate, propylene glycol dimethacrylate, and bisacrylates of oxyethylated bisphenol A derivatives. Also suitable are the low molecular weight, urethane group containing acrylates and methacrylates obtained by reaction with bivalent or polyvalent isocyanates.

For lithographic and printing plate purposes, the light sensitive composition described in U.S. Patent 4,289,838, the disclosure of which is hereby incorporated by reference, is particularly preferred. In broad terms, such compositions contain (a) solvent soluble, light sensitive diazonium components which can be any of the commonly used lithographic diazo compounds, or reaction or condensation products of such diazo compounds with agents therefor which do not materially impair the light sensitivity of the diazo, and (b) certain unsaturated monomers which are the products of about two equivalents of an organic di-isocyanate with about one equivalent of an active hydrogen containing organic compound such as an alcohol or amine, preferably a polyol, which products are subsequently reacted with an unsaturated compound which contains an active hydrogen, preferably in a hydroxyl group, available to react with the remaining isocyanate groups. The present invention, however, is not limited thereto.

In accordance with the conventional practice, the photoinitiator employed in combination with the photocurable composition can be benzophenone or other conventional agents. Preferably, as described in our copending application No. 60/3078/02 filed of even date herewith, the composition photoinitiator includes dibenzoyl benzene. The two benzoyl moieties can be positioned meta to one another or preferably are para to one another. The dibenzoyl benzene is an effective photoinitiator and is substantially more resistant to sublimation when supported on a substrate than benzophenone. Surprisingly the dibenzoyl benzene photo-initiator also causes the photocuring of the light sensitive composition to occur more rapidly.

According to the present invention para-di [4-dimethylamino-benzoyl] benzene is provided.

Also according to the present invention a light sensitive composition comprising a photocurable composition in combination with a photoinitiator is characterised in that the said photoinitiator comprises para-di [4-dimethylamino-benzyl] benzene.

It has been found that particularly fast photocuring is achieved when the dibenzoyl benzene is used in combination with the photoinitiator of the present invention, namely para-di[4-dimethylaminobenzoyl] benzene, as the photoinitiator system. To our knowledge, the para-di[4-dimethylaminobenzoyl] benzene is a new compound and the method by which it is produced is described in the examples below.

The quantity of photoinitiator in the light sensitive composition is conventional. The amount on a weight basis of benzophenone or dibenzoyl benzene is generally the same as the amount that benzophenone has been employed in the past and the amount of through-cure photoinitiator para-di[4-dimethylaminobenzoyl] benzene is generally the same as through-cure photoinitiators in conventional compositions.

The photocurable compositions can further contain fillers, binders, polymerization inhibitors, dyestuffs, color couplers, plasticizers, adhesion promotors, oxygen absorbing agents, and the like in varying quantities, in accordance with compositions well known in the art.

In order to further illustrate the invention, various examples are set forth below. In these examples, as throughout this specification and claims, all parts and percentages are by weight and all temperatures are in degrees centigrade unless otherwise specified.

Example 1

This example describes the preparation of the new compound para-di[4-dimethylaminobenzoyl] benzene.

To a one liter flask were added 206 ml of N,N-dimethylaniline and 56 g of anhydrous aluminum chloride. The contents of the flask were heated slowly to reflux at which time 40 g of terephthaloyl chloride dissolved in 345 ml of N,N-dimethylaniline was added over a period of one to two hours while maintaining reflux. The contents of the flask were thereafter refluxed for a further three hours at which time the flask was cooled to room temperature and the reaction mixture poured into a mixture of 8 liters of water and 2 liters of hydrochloric acid at 10° C, with vigorous agitation. The desired product precipitated and was worked up by filtration, washing with 2-3 liters of water, air-drying and two recrystallizations from ethyl alcohol. The para-di[4-dimethylaminobenzoyl] benzene obtained was a greenish gray material and had a melting point of 210-212° C.

| Elemental analysis: | C | H | O | N |
|---|---|---|---|---|
| Calc.: | 77.31 | 6.31 | 8.62 | 7.71 |
| Found: | 77.33 | 6.44 | 8.59 | 7.51 |

Example 2

In order to compare and contrast the speed and through-cure achieved using various photoinitiators, a

3

EP 0 372 778 A1

series of light sensitive coatings were prepared and hand-coated on a glass substrate. Each coating contained 94 parts of a light sensitive photocurable composition, and six parts of the initiator set forth in Table 1 below. Each coated glass plate was placed on a belt and conveyed past an ultraviolet lamp operated at 13.5 amps at a speed of 100 feet per minute. The lamp was positioned about 3 to 4 inches above the belt web and generated about 200 watts per linear inch. The resulting exposed plates were then evaluated first by touch to determine whether they were tacky, which indicated the degree of curing. The plates were also evaluated by the standard methyl ethyl ketone resistance test to determine whether this solvent dissolved the coating, the test being an indication degree of cross-linking or insolubilization of the coating and can be equated to the length of run of the printing plate on the lithographic press. The internal photoinitiator employed, belt speed, tackiness and methyl ethyl ketone (MEK) double rubs are set forth in table 2.

Table 1

|  | I | II | III | IV |
|---|---|---|---|---|
| p-dibenzoyl benzene | - | 5 | 5 | 1 |
| benzophenone | 5 | - | - | - |
| p-di(dimethylaminobenzoyl) benzene | - | - | 1 | 5 |
| Michler's ketone | 1 | 1 | - | - |

Table 2

| Photoinitiator | Tackiness | MEK Double Rubs |
|---|---|---|
| I | None | 4 |
| II | None | 4 |
| III | None | 23 |
| IV | None | 5 |

Various changes and modifications can be made in the products and process of the present invention without departing from the spirit and scope thereof. The various embodiments which have been set forth herein were for the purpose of further illustrating the invention but were not intended to limit it.

**Claims**

1. Para-di (4-dimethylaminobenzoyl) benzene
2. A light sensitive composition comprising a photocurable composition in combination with a photoinitiator characterised in that the said photoinitiator comprises p-di[4-dimethylaminobenzoyl] benzene.
3. A light sensitive composition of claim 2 wherein said photoinitiator further comprises dibenzoyl benzene.
4. A light sensitive composition of claim 3 wherein said dibenzoyl benzene is p-dibenzoyl benzene.
5. A light sensitive composition of any of claims 2 to 4 supported on a substrate.
6. A cured composition obtained by curing a light sensitive composition wherein said light sensitive composition is that of any of claims 2 to 5.

4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 069 555 (DU PONT DE NEMOURS) <br> * The whole document * <br> --- | 1 | G 03 F 7/031 <br> C 07 C 49/784 <br> C 07 C 225/22 |
| A | GB-A-1 223 463 (TIME INC.) <br> * Page 2, lines 12-17; claims * <br> ----- | 2-6 | |
| | | | **TECHNICAL FIELDS SEARCHED (Int. Cl.5)** <br><br> G 03 F 7/00 <br> C 07 C |

The present search report has been drawn up for all claims

| Place of search | Date of completion of the search | Examiner |
|---|---|---|
| THE HAGUE | 08-03-1990 | LUDI M.M.B. |

EPO FORM 1503 03.82 (P0401)